# EUROPÄISCHE PATENTSCHRIFT

(11) **EP 3 287 105 B1**
(45) Veröffentlichungstag und Bekanntmachung des Hinweises auf die Patenterteilung: **27.02.2019**
(21) Anmeldenummer: 16185835.2
(22) Anmeldetag: 26.08.2016
(51) Int. Cl.: A61F 9/008

(54) **LASERSYSTEM ZUM DURCHFÜHREN OPHTHAMOLOGISCHER EINGRIFFE**
LASER SYSTEM FOR CARRYING OUT OPHTHALMOLOGICAL PROCEDURES
SYSTÈME LASER DESTINÉ À ÉXÉCUTER DES INTERVENTIONS OPHTALMOLOGIQUES

(43) Veröffentlichungstag der Anmeldung: 28.02.2018
(73) Patentinhaber: Fritz Ruck Ophthalmologische Systeme GmbH, 52249 Eschweiler (DE)
(72) Erfinder: Perpeet, Markus, 52249 Eschweiler (DE)
(74) Vertreter: Schwarz & Partner Patentanwälte OG

(56) Entgegenhaltungen:
- WO-A1-2014/144697
- WO-A1-2016/137560
- WO-A1-2016/156760
- US-A1- 2010 130 966

## Beschreibung

Lasersystem zum Durchführen ophthamologischer Eingriffe mit einem Pulslaser zur Abgabe von Laserpulsen, einer Fokussieroptik zum Erzeugen wenigstens eines Brennpunkts im vorderen Bereich eines Auges eines Patienten, eine Ablenkeinrichtung zum Variieren der Position des Brennpunkts im vorderen Bereich des Auges des Patienten, und einer Steuereinrichtung zur Steuerung der Ablenkeinrichtung.

Aus der Druckschrift WO 2011/059958 A2 ist ein Lasersystem zum Durchführen von ophthamologischen Eingriffen bekannt, welches Lasersystem eine Fokussieroptik, eine Ablenkeinrichtung und einen Pulslaser zur Abgabe von Laserpulsen umfasst, wobei der Pulslaser zur Abgabe von Laserpulsen mit einer Pulsdauer im femto Sekundenbereich ausgebildet ist. Solche Pulslaser werden auch als Femto-Pulslaser bezeichnet. Durch den Einsatz von Femto-Pulslasern bei ophthamologischen Eingriffen ergeben sich gemäß der Druckschrift WO 2011/059958 A2 vielerlei Vorteile gegenüber Lasersystemen, die Pulslaser zum Durchführen von ophthamologischen Eingriffen beinhalten, die Laserpulse mit einer Pulsdauer im nano oder pico Sekundenbereich abgeben. Solche Laser werden auch als Nano-Pulslaser bzw. Pico-Pulslaser bezeichnet. Aufgrund der geringeren Pulsdauer der Laserpulse eines Femto-Pulslasers wird der Energieeintrag ins Gewebe verringert, wodurch die Präzision und die Steuerbarkeit des Eingriffs verbessert und bedingt dadurch das Risiko eines ungewollten Schadens von Gewebe minimiert wird. Aufgrund dessen hat sich in der Augenheilkunde der Einsatz von Femto-Pulslasern durchgesetzt.

Bei dem Einsatz von Femto-Pulslasern hat sich aber als nachteilig erwiesen, dass sich aufgrund des geringen Energieeintrags ins Gewebe eine Bestrahlungszeit auf das Gewebe erhöht, wodurch die Dauer von Eingriffen am Auge höher ist.

Es ist die Aufgabe der vorliegenden Erfindung ein Lasersystem für ophthamologische Eingriffe bereit zu stellen, bei dem die Dauer des Eingriffs am Patienten verringert wird und welches schnell und einfach zu handhaben ist.

Die Aufgabe wird erfindungsgemäß dadurch gelöst, dass der Pulslaser eine Pulsdauer im nano Sekundenbereich aufweist und dass das Lasersystem als Handheld-Gerät ausgebildet ist.

Infolge wird bei der vorliegenden Erfindung mit dem Vorurteil der Fachwelt gebrochen, dass Pulslaser, die Laserpulse mit einer Pulsdauer im nano Sekundenbereich abgeben, schlecht bis gar nicht bei ophthamologischen Eingriffen einsetzbar sind. Durch die längere Pulsdauer erhöht sich der Energieeintrag ins Gewebe, wodurch mittels Nano-Pulslaser gegenüber Femto-Pulslaser Eingriffe am Auge schneller vollzogen werden können. Für einen Schnitt im vorderen Bereich des Auges benötigt ein Femto-Pulslaser beispielsweise vier Sekunden, wohingegen ein Nano-Pulslaser nur Millisekunden benötigt. Hierdurch wird nicht nur Zeit eingespart und somit der Eingriff für den Patienten erträglicher, sondern es ermöglicht das Lasersystem als Handheld-Gerät auszubilden, da auf Systeme, die das Auge fixieren oder Systeme die das Auge fortwährend Tracken und Scannen verzichtet werden kann. Eine Fixierung des Auges während des Eingriffs birgt ein nicht unerhebliches Risiko, da die Fixierung des Auges zu einer Verzerrung des Auges führen kann, wodurch es zu Komplikationen während des Laserns des Gewebes kommen kann oder im schlimmsten Fall der operative Eingriff ein schlechtes Resultat zur Folge hat. Durch das Tracken bzw. das Scannen des Auges wird dieser Nachteil zwar vermieden, dennoch sind solche System sehr teuer.

Darüber hinaus ist durch den Einsatz eines Nano-Pulslaserers der Vorteil erhalten, dass das Lasersystem kostengünstiger ist, als ein Lasersystem mit einem Femto-Pulslaser.

Vorteilhaft wird so ein Lasersystem zum Durchführen einer Anterior-Kapsulotomie oder einer Posterior-Kapsulotomie am frei beweglichen Auge im vorderen Teil des Auges eingesetzt. Durch die Ausbildung des Lasersystems als Handheld-Gerät ist dabei der Vorteil erhalten, dass im selben Operationsbereich bzw. Operationsraum sowohl die Kapsulotomie, als auch weitere Eingriffe am Auge vorgenommen werden können. So kann zum Beispiel eine an die Kapsulotomie anschließende Fragmentierung der Augenlinse mit dem gleichen Lasersystem erfolgen oder auch durch Phakoemulsifikation. Das Verlagern des Patienten zwischen zwei Operationsschritten wird durch die einfache Handhabung und die Kompaktheit des Handheld-Geräts zur Gänze vermieden, wodurch die Möglichkeit von Komplikationen verringert wird und der Eingriff für den Patienten noch erträglicher ist.

Bevorzugt weist der Pulslaser eine Vorrichtung auf, welche zum Verändern einer Frequenz der Laserpulse ausgebildet ist, um auch am hinteren Teil des Auges Operationen durchzuführen. Diese Vorrichtung kann mechanische und/oder elektrische Komponenten aufweisen. Hierdurch ist der Vorteil erhalten, dass auf zusätzliches Gerät verzichtet werden kann und mit einem Gerät sehr viele verschiedene Eingriffe vollzogen werden können.

In einer weiteren Ausführungsvariante ist der Nano-Pulslaser in einem zum Handheld-Gerät extern angeordneten Gerät untergebracht, wobei die Laserimpulse über Faseroptik, zum Beispiel Glasfaserleiter, in das Handheld-Gerät übertragen werden.

Weitere vorteilhafte Ausführungsvarianten des erfindungsgemäßen Lasersystems zum Durchführen von ophthamologischen Eingriffen werden in weiterer Folge anhand der Figuren näher erläutert.
Figur 1 zeigt eine Ausführungsvariante eines erfindungsgemäßen Lasersystems in einer perspektivischen Ansicht.
Figur 2 zeigt einen Strahlengang des Lasersystems gemäß Figur 1 in einer schematischen Ansicht.

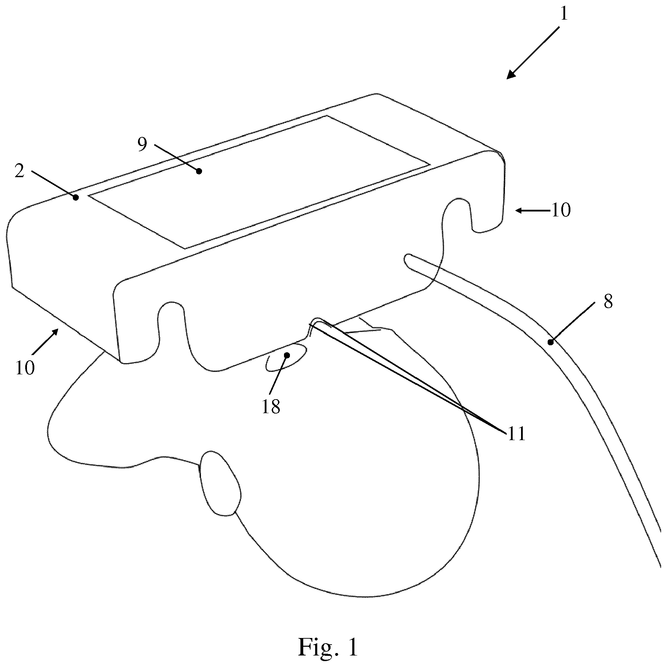

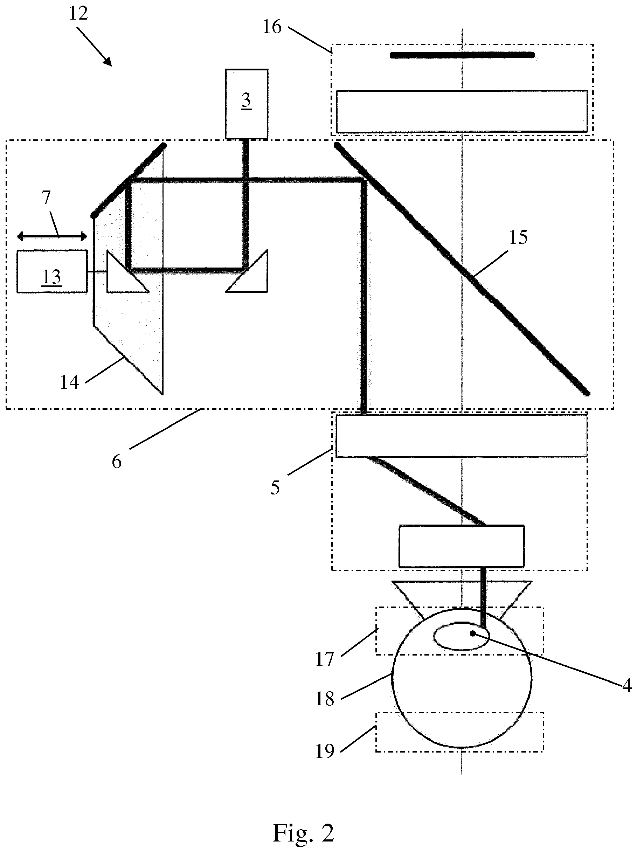

Figur 1 zeigt eine Ausführungsvariante eines erfindungsgemäßen Lasersystems 1 eingebaut in einem Gehäuse 2 während des Durchführens eines ophthamologischen Eingriffs an einem Auge 18 oder beiden Augen eines Patienten. Das Lasersystem 1 ist als Handheld-Gerät ausgebildet und kann mittels eines Kabels 8 zur Energieversorgung des Lasersystems 1 an eine Netzsteckdose angeschlossen werden. Das Lasersystem 1 weist einen Nano-Pulslaser 3, eine Fokussieroptik 5, eine Ablenkeinrichtung 6 und eine nicht dargestellte Steuereinheit auf. Der Nano-Pulslaser 3, die Fokussieroptik 5 und die Ablenkeinrichtung 6 sind schematisch in Figur 2 dargestellt. Darüber hinaus weist das Lasersystem 1 einen Touchscreen 9, nicht dargestellte Beleuchtungsmittel, Handgriffe 10 und Auflagepunkte 11 zum Auflegen des Handheld-Geräts auf den Patienten auf.

Figur 2 zeigt einen Strahlgang 12 des Lasersystems 1 gemäß Figur 1 in einer schematischen Ansicht. Ein von dem Nano-Pulslaser 3 erzeugter Laserimpuls wird über die Ablenkeinrichtung 6 und die Fokussieroptik 5 in einem Brennpunkt am zu operierenden Gewebe fokussiert, wobei mittels der Ablenkeinrichtung 6 eine Position des Brennpunkts am zu operierenden Gewebe variiert werden kann. Die Ablenkeinrichtung 6 weist dazu einen Elektromotor 13 auf, dessen Position gegenüber einem kegelstumpfförmigen Spiegel 14 mittels eines nicht dargestellten Linearantriebs entlang des Doppelpfeils 7 veränderbar ist. Der Nano-Pulslaser 3 ist ausgebildet, Eingriffe am vorderen Bereich 17 des Auges 18 durchzuführen, wobei der Nano-Pulslaser 3 vorteilhaft zum Abgeben von Laserimpulsen im Infrarotbereich ausgebildet ist. Ferner weist die Ablenkeinrichtung 6 einen dichroitischen Spiegel 15 und das Lasersystem 1 weist einen Bildsensor 16 auf. Der Nano-Pulslaser 3, der Elektromotor 13, der Linearantrieb, die Beleuchtungsmittel und der Touchscreen 9 sind mittels einer nicht dargestellten Steuereinheit zum Kommunizieren verbunden. Zweckmäßig sind die Beleuchtungsmittel durch LEDs gebildet und so gegenüber dem Auge 18 ausgerichtet, dass das Auge 18 im Lichtkegel der Beleuchtungsmittel liegt.

In weiterer Folge wird die Durchführung eines ophthamologischen Eingriffs mit dem Lasersystem 1 gemäß Figur 1 näher beschrieben. Nachdem sich der Patient in eine insbesondere waagrechte Operationslage begeben hat, wird durch einen Operateur das Handheld-Gerät auf das Gesicht des Patienten aufgelegt, wobei sich das Handheld-Gerät über die Auflagepunkte 11 am Gesicht, insbesondere an der Nase des Patienten, abstützt. Die Auflagepunkte 11 sind dabei vorteilhaft aus einem weichen Material gebildet, beispielsweise einem Elastomer. Auf dem Touchscreen 9 kann der Operateur auswählen, welche Operation durchgeführt wird. So kann zum Beispiel mit dem Handheld-Gerät eine Anterior-Kapsulotomie oder eine Posterior-Kapsulotomie durchgeführt werden. Im vorliegenden Fall wählt der Operateur zum Beispiel eine Anterior-Kapsulotomie aus. Mittels des Bildsensors 16 kann der Operateur über den Touchscreen 9 die Fokussieroptik 5 genau über dem Auge 18 des Patienten ausrichten. Über die Betätigung eines auf dem Gehäuse 2 angebrachten nicht dargestellten Schalters oder mittels Auswahl eines Icons am Touchdisplay 9 wird die Kapsulotomie gestartet, wobei durch den hohen Energieeintrag des Nano-Lasers 3 und eine daraus resultierende kurze Bestrahlungszeit des Gewebes des Kapselsacks des Auges 18 auf eine Fixierung, ein Tracken und/oder Scannen des Auges 18 verzichtet werden kann. Nachdem der Kapselsack geöffnet wurde, kann das Handheld-Gerät wieder abgenommen werden und beiseite gelegt werden. Nun kann mit der eigentlichen Operation am Auge 18 begonnen werden. Zum Beispiel dem Durchführen einer Fragmentierung einer Linse 4 mittels Phakoemulsifikation.

In einer weiteren Ausführungsvariante ist das Lasersystem 1 zur Fragmentierung von der Linse 4 des Auges 18 ausgebildet. Hierdurch ist der Vorteil erhalten, dass mit demselben Gerät zwei Operationsschritte durchgeführt werden können und sich die Eingriffsdauer noch einmal minimiert.

In einer weiteren Ausführungsvariante kann die Impulsfrequenz der Laserimpulse moduliert werden. Hierdurch ist der Vorteil erhalten, dass auch Eingriffe im hinteren Bereich 19 des Auges 18 vollzogen werden können.

## Patentansprüche

1. Lasersystem (1) zum Durchführen ophthalmologischer Eingriffe mit
einem Pulslaser (3) zur Abgabe von Laserpulsen, wobei der Pulslaser (3) eine Pulsdauer im nano Sekundenbereich aufweist,
einer Fokussieroptik (5) zum Erzeugen wenigstens eines Brennpunkts im vorderen Bereich (17) eines Auges (18) eines Patienten,
eine Ablenkeinrichtung (6) zum Variieren der Position des Brennpunkts im vorderen Bereich (17) des Auges (18) des Patienten, und
einer Steuereinheit zur Steuerung der Ablenkeinrichtung (6),
**dadurch gekennzeichnet, dass** das Lasersystem (1) in einem Gehäuse (2) des Lasersystems (1) ausgebildet ist, um ein Handheld-Gerät zu bilden, wofür das Gehäuse (2) Handgriffe (10) und Auflagepunkte (11) aufweist, welche Auflagepunkte (11) während des ophthalmologischen Eingriffs am Gesicht und insbesondere an der Nase des Patienten aufliegen.

2. Lasersystem (1) nach Anspruch 1, **dadurch gekennzeichnet, dass** das Lasersystem (1) zum Durchführen einer Anterior-Kapsulotomie oder einer Posterior-Kapsulotomie am frei beweglichen Auge (18) ausgebildet ist.

3. Lasersystem (1) nach Anspruch 1 oder 2, **dadurch gekennzeichnet, dass** der Pulslaser (3) eine Vorrichtung aufweist, welche zum Verändern einer Frequenz der Laserpulse ausgebildet ist, um im hinteren Bereich (19) des Auges (18) Operationen durchzuführen.

4. Lasersystem (1) nach einem der Ansprüche 1 bis 3, **dadurch gekennzeichnet, dass** das Lasersystem (1) zur Fragmentierung einer Linse (4) des Auges (18) ausgebildet ist.

5. Lasersystem (1) nach einem der Ansprüche 1 bis 4, **dadurch gekennzeichnet, dass** das Lasersystem (1) einen Touchscreen (9) aufweist.

6. Lasersystem (1) nach einem der Ansprüche 1 bis 5, **dadurch gekennzeichnet, dass** das Lasersystem (1) zumindest ein Beleuchtungsmittel aufweist, welches zum Beleuchten des Auges (18) während dem ophthalmologischen Eingriffs ausgebildet ist.

7. Lasersystem (1) nach einem der Ansprüche 1 bis 6, **dadurch gekennzeichnet, dass** das Lasersystem (1) zur Energieversorgung ein Kabel (8) aufweist, über das es an eine Netzsteckdose anschließbar ist.

## Claims

1. A laser system (1) for carrying out ophthalmological interventions having a pulsed laser (3) for emitting laser pulses, wherein the pulsed laser (3) has a pulse duration in the nanosecond range,
a focussing optics (5) for generating at least one focal point in the front area (17) of the eye (18) of a patient,
a deflection device (6) for varying the position of the focal point in the front area (17) of the eye (18) of the patient, and
a control unit for controlling the deflection device (6),
**characterized in that** the laser system (1) is situated within a housing (2) of the laser system (1) in order to form a handheld device, for which the housing (2) has handles (10) and support points (11), which support points (11) rest on the face and in particular on the nose of the patient during the ophthalmological intervention.

2. The laser system (1) according to claim 1, **characterized in that** the laser system (1) is configured to carry out an anterior capsulotomy or a posterior capsulotomy on the freely movable eye (18).

3. The laser system (1) according to claim 1 or 2, **characterized in that** the pulsed laser (3) has a device, which is configured to change a frequency of the laser pulses in order to carry out surgical interventions in the rear area (19) of the eye (18).

4. The laser system (1) according to any of claims 1 to 3, **characterized in that** the laser system (1) is configured to fragmentize a lens (4) of the eye (18).

5. The laser system (1) according to any of claims 1 to 4, **characterized in that** the laser system (1) has a touch screen (9).

6. The laser system (1) according to any of claims 1 to 5, **characterized in that** the laser system (1) has at least one illumination means, which is configured to illuminate the eye (18) during the ophthalmological intervention.

7. The laser system (1) according to any of claims 1 to 6, **characterized in that** the laser system (1) has a cable (8) for energy supply, via which it may be connected to a power socket.

## Revendications

1. Système à laser (1) destiné à exécuter des interventions ophtalmologiques, comportant
un laser pulsé (3) pour émettre des impulsions laser, le laser pulsé (3) présentant une durée d'impulsion dans la plage de la nanoseconde,
une optique de focalisation (5) pour générer au moins un foyer dans la zone avant (17) d'un oeil (18) d'un patient,
un moyen de déviation (6) pour faire varier la position du foyer dans la zone avant (17) de l'oeil (18) du patient, et
une unité de commande pour commander le moyen de déviation (6), **caractérisé en ce que** le système à laser (1) est réalisé dans un boîtier (2) du système à laser (1) pour former un appareil tenu à la main, ce pourquoi le boîtier (2) comprend des poignées (10) et des points d'appui (11), lesdits points d'appui (11) prenant appui contre le visage et en particulier contre le nez du patient pendant l'intervention ophtalmologique.

2. Système à laser (1) selon la revendication 1, **caractérisé en ce que** le système à laser (1) est réalisé pour exécuter une capsulotomie antérieure ou une capsulotomie postérieure sur l'oeil (18) librement mobile.

3. Système à laser (1) selon la revendication 1 ou 2, **caractérisé en ce que** le laser pulsé (3) comprend un dispositif qui est réalisé pour varier une fréquence des impulsions laser pour exécuter des opérations dans la zone arrière (19) de l'oeil (18).

4. Système à laser (1) selon l'une des revendications 1 à 3, **caractérisé en ce que** le système à laser (1) est réalisé pour la fragmentation d'une lentille (4) de l'oeil (18).

5. Système à laser (1) selon l'une des revendications 1 à 4, **caractérisé en ce que** le système à laser (1) présente un écran tactile (9).

6. Système à laser (1) selon l'une des revendications 1 à 5, **caractérisé en ce que** le système à laser (1) comprend au moins un moyen d'éclairage qui est réalisé pour éclairer l'oeil (18) pendant l'intervention ophtalmologique.

7. Système à laser (1) selon l'une des revendications 1 à 6, **caractérisé en ce que** le système à laser (1) présente un câble (8) pour l'alimentation en énergie, via lequel il peut être branché à une prise du secteur.
